# EUROPEAN PATENT SPECIFICATION

(11) **EP 3 015 100 B1**
(45) Date of publication and mention of the grant of the patent: **25.09.2019**
(21) Application number: 14816733.1
(22) Date of filing: 27.06.2014
(51) Int. Cl.: A61K 8/81, A61K 8/34, A61K 8/35, A61K 8/37, A61K 8/39, A61K 8/60, A61K 8/92, A61Q 19/04

(54) **COSMETIC**
KOSMETIKUM
PRODUIT COSMÉTIQUE

(30) Priority: 28.06.2013 JP 2013137449
(43) Date of publication of application: 04.05.2016
(73) Proprietor: Kao Corporation, Tokyo 103-8210 (JP)
(72) Inventor: KATSUKURA, Hiroaki, Odawara-shi Kanagawa 250-0002 (JP)
(74) Representative: Hoffmann Eitle
(86) International application number: PCT/JP2014/067212
(87) International publication number: WO 2014/208738

(56) References cited:
- EP-A1- 0 576 188
- EP-A1- 1 481 663
- EP-A1- 1 570 836
- JP-A- H0 656 630
- JP-A- 2001 505 570
- JP-A- 2007 523 869
- JP-A- 2010 132 566
- US-A1- 2007 122 364
- US-A1- 2007 183 994
- Chemical-Navi.Com: "NIKKOL Hexaglyn 1-SV(Polyglyceryl-6 Stearate) | Product Search <chemical-navi.com>", , 31 December 2010 (2010-12-31), XP055383006, Retrieved from the Internet: URL:https://www.chemical-navi.com/english/ product_search/detail106.html [retrieved on 2017-06-20]

## Description

### [Field of the Invention]

The present invention relates to a cosmetic composition comprising a tanning agent.

### [Background of the Invention]

A self-tanning cosmetic composition is a cosmetic composition which contains a tanning agent such as dihydroxyacetone and can make tanned skin like suntanned skin by causing Maillard reaction between the tanning agent and amino acids on the skin.

Generally, it takes some time for a tanning agent to develop color, and it takes 5 or 6 hours to exhibit sufficient tanning effect. Immediately after application to the skin, a self-tanning cosmetic composition is transparent and therefore it is not possible to judge whether the cosmetic composition has been applied evenly. If a self-tanning cosmetic composition is not applied to the skin evenly, a problem such as orange coloring of the skin, or mottled appearance of skin can occur. Therefore, the viscosity of the tanning cosmetic composition is adjusted to a certain level or more by using a thickening agent or the like so that the tanning cosmetic composition can be applied evenly.

Meanwhile, because the tanning agent is an unstable substance having high reactivity, securing storage stability is a big problem. For example, it is known that dihydroxyacetone, one of the tanning agents, loses its tanning effect at a pH of 7 or more due to isomerization and condensation.

Therefore, the self-tanning cosmetic composition needs to be adjusted to acidic side, and then there is a problem as follows: carboxyvinyl polymer, which is widely used in cosmetic field because of its inexpensiveness and high thickening effect, was not to be utilized since carboxyvinyl polymer can exhibit the thickening effect only within a limited pH range, but not within a pH range of weak acid or lower.

To solve the problems as described above, for example, Patent Literature 1 proposes a transparent self-suntanning aqueous gel having an enhanced storage stability by containing a water-soluble or water-dispersible acrylamido-2-methylpropanesulfonic acid polymer. Further, Patent Literature 2 proposes a stable cosmetic composition having a PH of from 3.5 to 4.5 and containing a self-tanning agent, a polymer and a polyhydric alcohol humectant. Furthermore, Patent Literature 3 discloses a cosmetic composition comprising a α-hydroxy substituted ketone or aldehyde such as dihydroxyacetone, a polyacrylamide and a pharmaceutically acceptable carrier providing an enhanced color intensity. Furthermore, EP1481663 discloses self-tanning compositions.

Patent Literature 1: JP-B-3876266
Patent Literature 2: JP-A-2007-526216
Patent Literature 3: EP 0 576 188 A1

### [Summary of the Invention]

The present invention relates to a cosmetic composition comprising the following components (A) to (E), and having a viscosity of from 3,000 to 12,000 mPa·s:
(A) 0.3 to 1.5 mass% of a copolymer or crosspolymer which comprises, as a constituent unit, a combination of either an acrylic acid salt and 2-acrylamido-2-methylpropanesulfonic acid or a salt thereof, or of a hydroxyethyl acrylate and 2-acrylamido-2-methylpropanesulfonic acid or a salt thereof; polyacrylamide; or a mixture thereof,
(B) 0.3 to 3 mass% of a polyglycerol fatty acid ester;
(C) 13 to 31 mass% of a polyhydric alcohol;
(D) 2 to 18 mass% of a liquid oil having a melting point of 30°C or lower; and
(E) 2 to 8 mass% of a tanning agent,
wherein the component (D) comprises 70 mass% or more of a fatty acid triglyceride having at least one fatty acid group having 5 to 22 carbon atoms.

Also, the present invention relates to use of the cosmetic composition described above as a self-tanning cosmetic composition.

Further, the present invention relates to a method for adjusting a color tone of the skin, comprising topically applying the cosmetic composition described above to the skin.

### [Detailed Description of the Invention]

In the invention described in Patent Literature 1, storage stability is enhanced by incorporating a water-soluble or water-dispersible acrylamido-2-methylpropanesulfonic acid copolymer, but in a case of a self-suntanning aqueous gel comprising an oil agent in a small amount, moisturizing effect is unsufficient, which leads to the problem of bringing a user dry feeling on application. In the invention described in Patent Literature 2, storage stability is secured by making the cosmetic composition have a viscosity of a certain level or more, but high viscosity leads to the problem of decreasing tanning speed. Further, when a large amount of a moisturizing agent or an oil agent is incorporated, stickiness feeling becomes stronger, and additionally it takes too much time to dry, which results in the problem of difficulty in even application.

Therefore, the present invention provides a cosmetic composition having excellent storage stability and excellent moisturizing effect as well as excellent tanning speed and evenness of tanning.

As a result of an earnest study, the present inventor found that when a specific acrylic polymer, a polyglycerol fatty acid ester, a polyhydric alcohol, a liquid oil, and a tanning agent are used in combination, a cosmetic composition can be obtained which, regardless of low viscosity, very surprisingly has good moisturizing property, excellent storage stability of the tanning agent, high tanning speed, and excellent even applicability, and thus completed the present invention.

The cosmetic composition of the present invention has low viscosity as well as excellent storage stability of the tanning agent, and high moisturizing effect, and can make tanned skin having natural tint. Further, the cosmetic composition provides excellent tanning speed and evenness of tanning, and brings excellent feeling upon application without stickiness feeling.

The constitution of the present invention will now be described in detail.

The cosmetic composition of the present invention comprises the components (A) to (E) described above.

The component (A) in the present invention is a copolymer or crosspolymer which comprises, as a constituent unit, a combination of either an acrylic acid salt and 2-acrylamido-2-methylpropanesulfonic acid or a salt thereof, or of a hydroxyethyl acrylate and 2-acrylamido-2-methylpropanesulfonic acid or a salt thereof; polyacrylamide; or a mixture thereof.

In view of storage stability of the cosmetic composition, evenness of tanning, tanning speed and prevention of stickiness feeling, it is preferable to use one or two or more selected from the group consisting of polyacrylamide, a copolymer of an acrylic acid salt and acryloyldimethyltaurate, and a copolymer of hydroxyethyl acrylate and acryloyldimethyltaurate; it is more preferable to use one or two or more selected from the group consisting of polyacrylamide and a copolymer of hydroxyethyl acrylate and acryloyldimethyltaurate; and it is even more preferable to use a copolymer of hydroxyethyl acrylate and acryloyldimethyltaurate. Specifically, it is even more preferable to use one or two or more selected from the group consisting of (hydroxyethyl acrylate/sodium acryloyldimethyltaurate) copolymer, (sodium acrylate/sodium acryloyldimethyltaurate) copolymer, and polyacrylamide.

For these, SEPINOV EMT10, SIMULGEL NS, SIMULGEL FL, and SIMULGEL S as (hydroxyethyl acrylate/sodium acryloyldimethyltaurate) copolymer, SIMULGEL EG as (sodium acrylate/sodium acryloyldimethyltaurate) copolymer, and SEPIGEL305 and SEPIGEL501 as polyacrylamide are commercially available from SEPPIC.

In view of storage stability of the cosmetic composition, evenness of tanning, tanning speed and prevention of stickiness feeling, the content of the component (A) in the cosmetic composition is 0.3 mass% or more, and preferably 0.5 mass% or more, and is 1.5 mass% or less. The specific range of the content is from 0.3 to 1.5 mass%, and preferably from 0.5 to 1.5 mass%.

As the fatty acid constituting the polyglycerol fatty acid ester of the component (B) in the present invention, a plurality kinds of fatty acids may be mixed and used, and a mixture of fatty acids such as a saturated synthetic fatty acid, a natural animal fatty acid, and a fatty acid derived from vegetable oil and fat may be used. As the fatty acid constituting the polyglycerol fatty acid ester, for example, a saturated or unsaturated fatty acid having 12 to 22 carbon atoms can be used, and among these, a saturated or unsaturated fatty acid having 14 to 18 carbon atoms is preferable. Examples thereof include lauric acid, myristic acid, palmitic acid, stearic acid and behenic acid, and one or two or more of these can be used. Among these, particularly, one or two or more selected from the group consisting of myristic acid, palmitic acid and stearic acid are preferably used because the resulting cosmetic composition exhibits low irritation and thus provides high safety when applied to the skin.

The condensation degree of glycerin for the polyglycerol fatty acid ester (B) is preferably from 2 to 10, and more preferably from 6 to 10. The esterification degree of the polyglycerol fatty acid ester (B) (percentage (%) of esterified fatty acids relative to all the hydroxyl groups of polyglycerin) is preferably from 20 to 75%, and more preferably from 30 to 55%.

The component (B) is preferably a polyglycerol fatty acid ester which is an ester of polyglycerin having a condensation degree of from 2 to 10 and a saturated or unsaturated fatty acid having 12 to 22 carbon atoms and has an esterification degree of from 20 to 75%.

Specific examples of the polyglycerol fatty acid ester preferably used include tri-, tetra-, penta-, hexa- or hepta-fatty acid ester of hexaglycerol; and tri-, tetra-, penta-, hexa-, hepta-, octa-, nona-, deca- or undeca-fatty acid ester of decaglycerol.

Specific examples of commercially available products include Sunsoft Q14Y-C (polyglyceryl-10 myristate, HLB=14.5), Q142Y-C (polyglyceryl-10 dimyristate, HLB=12.3), Q18Y-C (polyglyceryl-10 stearate, HLB=17.5), Q18S-C(polyglyceryl-10 stearate, HLB=12.0), A14E-C (polyglyceryl-10 myristate, HLB=13.0), A18E-C (polyglyceryl-5 stearate, HLB=13.0) (manufactured by Taiyo Kagaku Co., Ltd.), S face M1001 (polyglyceryl-10 myristate, HLB=14.8), S1001(polyglyceryl-10 stearate, HLB=14.1) (manufactured by Sakamoto Yakuhin kogyo Co., Ltd.), NIKKOL Hexaglyn1-M (polyglyceryl-6 myristate, HLB=11.0), and 1-SV (polyglyceryl-6 stearate, HLB=9.0) (manufactured by Nikko Chemicals Co., Ltd.). It is preferable to use one or two or more selected from these commercially available products.

Preferably, the polyglycerol fatty acid ester (B) is a liquid polyglycerol fatty acid ester which is in a fluid state (including a paste state) at 25°C. In view of the storage stability and tanning effect of the cosmetic composition, the polyglycerol fatty acid ester (B) preferably has an HLB of 9 or more, more preferably an HLB of 10 or more, and even more preferably an HLB of 12 or more, and preferably has an HLB of 20 or less, more preferably an HLB of 18 or less, and even more preferably an HLB of 16 or less. The specific range of HLB is preferably from 9 to 20, more preferably from 10 to 18, and even more preferably from 12 to 16.

Here, HLB can be calculated by the following equation (Griffin equation):

HLB = 20(1-S/A)

wherein S represents the saponification value, and A represents the neutralization value of the fatty acid. When two or more of polyglycerol fatty acid esters are used, an HLB of the mixture thereof can be calculated as a weighted average of HLBs of each polyglycerol fatty acid esters.

The polyglycerol fatty acid ester is often obtained as a mixture, and in this case, the mixture is constituted of components having different glycerol condensation degrees and/or esterification degrees.

In view of storage stability of the cosmetic composition, evenness of tanning, tanning speed and prevention of stickiness feeling, the content of the polyglycerol fatty acid ester (B) in the cosmetic composition is 0.3 mass% or more, and preferably 0.5 mass% or more, and is 3 mass% or less, and preferably 1 mass% or less. The specific range of the content is from 0.3 to 3 mass%, preferably from 0.3 to 1 mass%, and more preferably from 0.5 to 1 mass%.

The polyhydric alcohol (C) used in the present invention is a compound having two or more hydroxyl groups in the molecule, and a di- to tetrahydric alcohol can be preferably used. Examples of the polyhydric alcohol include glycols such as ethylene glycol, diethylene glycol, triethylene glycol, polyethylene glycol (having average molecular weight of less than 650), propylene glycol, dipropylene glycol, polypropylene glycol (having average molecular weight of less than 650), isoprene glycol and 1,3-butylene glycol; glycerin, diglycerin, polyglycerin; and pentaerythritol. These polyhydric alcohols can be used singly or in any combinations of two or more.

Among these, one or two or more selected from the group consisting of ethylene glycol, diethylene glycol, propylene glycol, dipropylene glycol, 1,3-butylene glycol, glycerin, diglycerin and pentaerythritol can be preferably used, and one or two or more selected from the group consisting of dipropylene glycol, 1,3-butylene glycol, glycerin and diglycerin can be more preferably used, in view of solubility and usability.

The content of the polyhydric alcohol (C) in the cosmetic composition is 13 mass% or more, and preferably 16 mass% or more in view of storage stability of the cosmetic composition and evenness of tanning. The content is 31 mass% or less, and preferably 27 mass% or less in view of tanning speed. The specific range of the content is from 13 to 31 mass%, and preferably from 16 to 27 mass%. Because a cosmetic composition exhibiting good moist feeling without friction feeling or stickiness feeling can be provided, the range above mentioned is preferable.

The liquid oil (D) used in the present invention is an oil agent having a melting point of 30°C or lower. The oil agent is an oil agent having fluidity at 1 atm and 25°C. The method for measuring the melting point is according to the method 3 described in Japanese Standards of Cosmetic Ingredients.

In the present invention, in view of storage stability, tanning speed, and smooth feeling upon application, the liquid oil (D) comprises 70 mass% or more of a fatty acid triglyceride having at least one fatty acid group having 5 to 22 carbon atoms. As such a fatty acid triglyceride, a fatty acid triglyceride having at least two fatty acid groups having 5 to 22 carbon atoms is preferred, and a fatty acid triglyceride having three fatty acid groups having 5 to 22 carbon atoms is more preferred, in view of feeling upon application when applying to the skin, particularly, in view of light feeling and fresh feeling.

Examples of such a fatty acid triglyceride include vegetable oils such as olive oil, macadamia nut oil, meadowfoam oil, castor oil, safflower oil, sunflower oil, avocado oil, canola oil, apricot kernel oil, rice germ oil and rice bran oil; and synthetic triglyceride such as tricaproin, tricaprylin, triisostearin, trielaidin, trierucine, trilinolein, triolein, glyceryl tri(caprylate/caprate), glyceryl tri(caprylate/caprate/linoleate), and glyceryl tri(ricinoleate/caproate/caprylate/caprate), and those which are commercially available as a material for cosmetics or pharmaceuticals can be utilized. In the present invention, one or two or more of these can be selected and used.

Among these, preferred are one or more vegetable oils selected from the group consisting of olive oil, macadamia nut oil, meadowfoam oil, castor oil, safflower oil, sunflower oil, avocado oil, canola oil, apricot kernel oil, rice germ oil and rice bran oil; or a fatty acid triglyceride in which fatty acid residual groups constituting the fatty acid triglyceride have 6 to 10 carbon atoms, and more preferred are one or more fatty acid triglyceride selected from the group consisting of olive oil, macadamia nut oil and glyceryl tri(caprylate/caprate), in view of good storage stability, tanning speed and evenness of tanning.

In view of good storage stability, tanning speed, evenness of tanning, and smooth feeling upon application, the content of the fatty acid triglyceride above described in the liquid oil (D) is from 70 to 100 mass%, and preferably from 75 to 100 mass%. The component (D) is a liquid oil comprising 70 mass% or more of a fatty acid triglyceride having at least one fatty acid group having 5 to 22 carbon atoms, preferably a liquid oil comprising 70 mass% or more of a fatty acid triglyceride having at least two fatty acid groups having 5 to 22 carbon atoms, and more preferably a liquid oil comprising 70 mass% or more of a fatty acid triglyceride having three fatty acid groups having 5 to 22 carbon atoms, in the component (D).

The content of the liquid oil (D) in the cosmetic composition is 2 mass% or more, and preferably 5 mass% or more in view of tanning speed, evenness of tanning, and good moisturizing feeling, and is 18 mass% or less, and preferably 15 mass% or less in view of storage stability of the cosmetic composition and prevention of stickiness feeling. More specifically, the content is from 2 to 18 mass%, and preferably from 5 to 15 mass%.

In the present invention, a solid oil (F) can be used as an optional component in order to adjust viscosity or feeling, and it is preferably used in an amount of 25 mass% or less, more preferably from 0 to 20 mass% or less, even more preferably from 0 to 10 mass%, and even more preferably from 0 to 5 mass% based on the total amount of the liquid oil (D), in view of tanning speed and evenness of tanning.

In the present invention, the solid oil refers to an oil agent having a melting point higher than 30°C. Such an oil agent is in a state of no fluidity or low fluidity at 25°C, encompassing oil agents in a paste (semisolid) state. The method for measuring the melting point is according to the method 3 described in Japanese Standards of Cosmetic Ingredients.

Examples of the oil agent encompassed by the solid oil (F) include ester oils such as glyceryl trilanolate, soft lanolin acid, dipentaerythrityl fatty acid ester (dipentaerythrityl hexaoxystearate, etc.), hydrogenated castor oil isostearate, hydrogenated castor oil monohydroxystearate, glyceryl tri(caprylate/caprate/myristate/stearate), and myristyl lactate; hydrocarbons such as ceresin, paraffin wax, polyethylene powder, microcrystalline wax, and vaseline; oils and fats such as coconut oil, shea butter, beef tallow, hydrogenated oil, and horse fat; waxes such as carnauba wax, candelilla wax, rice bran wax, shellac, lanolin, and beeswax; fatty acids such as lauric acid, myristic acid, palmitic acid, stearic acid, behenic acid, and 12-hydroxystearic acid; and higher alcohols such as myristyl alcohol, cetyl alcohol, stearyl alcohol, behenil alcohol, cholesterol, and phytosterol.

The tanning agent (E) in the present invention is a component which can cause Maillard reaction with amino acids on the skin when applied to the skin to thereby make the skin brown-colored like suntanned skin, and specific examples thereof include dihydroxyacetone, glyceraldehyde, 2,3-dihydroxy-succindialdehyde, 2,3-dimethoxysuccindialdehyde, erythrulose, erythrose, 2-amino-3-hydroxy-succindialdehyde, and 2-benzylamino-3-hydroxy-succindialdehyde. One or two or more of these can be used.

Among them, it is preferred to contain dihydroxyacetone or erythrulose, and it is more preferred to contain two of dihydroxyacetone and erythrulose, in view of tanning effect and evenness of tanning. These are generally commercially available, and example thereof include DHAPlus, DHArapid (manufactured by Merck & Co., Inc.) and erythrulose (manufactured by DSN V.V.)

The content of the tanning agent in the cosmetic composition is 2 mass% or more, and preferably 3 mass% or more, and is 8 mass% or less, and preferably 5 mass% or less, in view of good tanning effect, evenness of tanning, and tanning speed. The specific range of the content is from 2 to 8 mass%, preferably from 2 to 5 mass%, and more preferably from 3 to 5 mass%.

The cosmetic composition of the present invention can comprise a polymer other than the component (A) such as a water soluble cationic polymer, an anionic polymer, a nonionic polymer, and an amphoteric polymer or a bipolar polymer, as long as the effect of the present invention is not impaired.

Specific examples of the cationic polymer include hydroxyethyl cellulose having an O-[2-hydroxy-3-(trimethylammonio)propyl]chloride group (polyquaternium-10), diethyl sulfate salt of (vinylpyrrolidone/dimethylaminomethylethyl methacrylate copolymer (polyquaternium-11), and methylvinylimidazolium chloride/vinylpyrrolidone copolymer.

Specific examples of the anionic polymer include carboxyvinyl polymer, carboxymethyl cellulose, carrageenan, xanthan gum, polystyrene sulfonate, agar, ghatti gum, karaya gum, pectin, alginate salt, acrylic acid/alkyl methacrylate copolymer, poly(acrylic acid), acrylic acid or methacrylic acid derivatives such as alkali metal salts and ammonium salts of acrylic acid or methacrylic acid, and hyaluronic acid or alkali metal salts thereof.

Specific examples of the nonionic polymer include cellulose ethers (hydroxybutylmethyl cellulose, hydroxypropyl cellulose, hydroxypropylmethyl cellulose, ethylhydroxyethyl cellulose, hydroxyethyl cellulose, etc.), propylene glycol alginate, polyacrylic acid or salts thereof, poly(ethylene oxide), polyvinyl alcohol, polyvinylpyrrolidone, hydroxypropyl guar gum, locust bean gum, guar gum, tamarind seed gum, amylose, hydroxyethyl amylose, starch and starch derivatives, and a mixture thereof.

Specific examples of the amphoteric polymer or the bipolar polymer include octylacrylamide/acrylate/butylaminoethyl methacrylate copolymer, polyquaternium-47 and polyquaternium-43.

Among these, preferred are xanthan gum, carrageenan, locust bean gum, guar gum and tamarind seed gum; more preferred are xanthan gum, locust bean gum and guar gum; and even more preferred is xanthan gum. One or two or more of these can be used.

The content of these polymers other than the component (A) in the cosmetic composition is preferably 0.01 mass% or more, more preferably 0.1 mass% or more, and even more preferably 0.15 mass% or more, and is preferably 5 mass% or less, more preferably 3 mass% or less, even more preferably 2 mass% or less, and even more preferably 1 mass% or less, in view of adjustment of feeling upon application and adjustment of viscosity. The specific range of the content is preferably from 0.01 to 5 mass%, more preferably from 0.1 to 3 mass%, even more preferably from 0.15 to 2 mass%, and even more preferably from 0.15 to 1 mass%.

In the present invention, an amphoteric surfactant, a nonionic surfactant, a gum substance, a propellant, an antiseptic, a chelating agent, an antioxidant, a pH adjuster, a colorant, a perfume, a powder, or the like can also be incorporated in addition to the essential components described above, as long as the effect of the invention is not impaired.

In view of tanning speed and evenness of tanning, the viscosity of the cosmetic composition of the present invention is from 3,000 to 12,000 mPa·s, is preferably 4,000 mPa·s or higher, and is preferably 9,000 mPa·s or lower. Specifically, the viscosity is preferably from 4, 000 to 9, 000 mPa·s.

Viscosity in the present invention is as measured with a B-type viscometer (Vismetron viscosity meter: model VS-A1 (manufactured by SHIBAURA SYSTEM CO., LTD.) under the conditions of 1 atm and 25°C.

Specifically, viscosity higher than 500 mPa·s and 10,000 mPa·s or lower is measured with a spindle of No. 3 at 12 rpm (rotation/minute) for 30 seconds, and viscosity higher than 10,000 and 20,000 mPa·s or lower is measured with a spindle of No. 4 at 12 rpm (rotation/minute) for 30 seconds.

In view of storage stability of the cosmetic composition and the effect of tanning, the pH value of the cosmetic composition of the present invention is preferably 7 or less, more preferably 6 or less, even more preferably 5 or less, and even more preferably 4 or less, and is preferably 3 or more. Specifically, the pH value is preferably 7 or less, more preferably from 3 to 6, even more preferably from 3 to 5, and even more preferably from 3 to 4. The pH value in the present invention is a value measured with a pH meter (manufactured by HORIBA, Ltd., model F-22) under the conditions of 1 atm and 25°C.

In view of storage stability of the cosmetic composition, the effect of tanning, moisturizing feeling, and fresh feeling upon application, the cosmetic composition of the present invention is preferably an emulsified composition, and more preferably an oil-in-water emulsified composition. In view of storage stability and fresh feeling upon application, the mass ratio of the oil phase to the water phase (oil phase/water phase) in the emulsified composition is preferably 0.01 or more, and more preferably 0.05 or more, and is preferably 0.4 or less, and more preferably 0.2 or less. The specific range of the mass ratio is preferably from 0.01 to 0.4, and more preferably from 0.05 to 0.2.

The water content in the emulsified composition is preferably 50 mass% or more, and more preferably 55 mass% or more, and is preferably 80 mass% or less, and more preferably 75 mass% or less, based on the whole composition. The specific range of the content is preferably from 50 to 80 mass%, and more preferably from 55 to 75 mass%.

The cosmetic composition of the present invention can be used as a self-tanning cosmetic composition, and it can be also utilized as a sunscreen cosmetic composition, a make-up base cosmetic composition or the like. The applicable form of the cosmetic composition of the present invention is a lotion or an emulsion form as well as a sheet, a spray or a mousse. When the cosmetic composition of the present invention is topically applied to the skin, the color tone of the skin can be adjusted.

In connection with the embodiments mentioned above, the present invention discloses the following cosmetic composition, use and method.

<1> A cosmetic composition comprising the following components (A) to (E), and having a viscosity of from 3,000 to 12,000 mPa·s:
   (A) 0.3 to 1.5 mass% of a copolymer or crosspolymer which comprises, as a constituent unit, a combination of either an acrylic acid salt and 2-acrylamido-2-methylpropanesulfonic acid or a salt thereof, or of a hydroxyethyl acrylate and 2-acrylamido-2-methylpropanesulfonic acid or a salt thereof; polyacrylamide; or a mixture thereof,
   (B) 0.3 to 3 mass% of a polyglycerol fatty acid ester;
   (C) 13 to 31 mass% of a polyhydric alcohol;
   (D) 2 to 18 mass% of a liquid oil having a melting point of 30°C or lower; and
   (E) 2 to 8 mass% of a tanning agent,
   wherein the component (D) comprises 70 mass% or more of a fatty acid triglyceride having at least one fatty acid group having 5 to 22 carbon atoms.
<2> The cosmetic composition according to <1>, wherein the component (A) is preferably one or two or more selected from the group consisting of polyacrylamide, a copolymer of an acrylic acid salt and acryloyldimethyltaurate, and a copolymer of hydroxyethyl acrylate and acryloyldimethyltaurate; more preferably one or two or more selected from the group consisting of polyacrylamide and a copolymer of hydroxyethyl acrylate and acryloyldimethyltaurate; and even more preferably a copolymer of hydroxyethyl acrylate and acryloyldimethyltaurate.
<3> The cosmetic composition according to <1> or <2>, wherein the component (A) is one or two or more selected from the group consisting of (hydroxyethyl acrylate/sodium acryloyldimethyltaurate) copolymer, (sodium acrylate/sodium acryloyldimethyltaurate) copolymer, and polyacrylamide.
<4> The cosmetic composition according to any of <1> to <3>, wherein a content of the component (A) in the cosmetic composition is 0.3 mass% or more, and preferably 0.5 mass% or more, and is 1.5 mass% or less.
<5> The cosmetic composition according to any of <1> to <4>, wherein the content of the component (A) in the cosmetic composition is 0.3 to 1.5 mass%, and preferably from 0.5 to 1.5 mass%.
<6> The cosmetic composition according to any of <1> to <5>, wherein the component (B) is an ester of polyglycerin preferably having a condensation degree of from 2 to 10, more preferably 6 to 10, and a saturated or unsaturated fatty acid preferably having 12 to 22 carbon atoms, more preferably 14 to 18 carbon atoms.
<7> The cosmetic composition according to any of <1> to <6>, wherein the component (B) is a polyglycerol fatty acid ester preferably having an esterification degree of from 20 to 75%, more preferably from 30 to 55%.
<8> The cosmetic composition according to any of <1> to <7>, wherein the component (B) is an ester of polyglycerin having a condensation degree of from 2 to 10 and a saturated or unsaturated fatty acid having 12 to 22 carbon atoms, and has an esterification degree of 20 to 75%.
<9> The cosmetic composition according to any of <1> to <8>, wherein the component (B) is a polyglycerol fatty acid ester preferably having an HLB of 9 or more, more preferably an HLB of 10 or more, and even more preferably an HLB of 12 or more, and preferably having an HLB of 20 or less, more preferably an HLB of 18 or less, and even more preferably an HLB of 16 or less.
<10> The cosmetic composition according to any of <1> to <9>, wherein the component (B) is a polyglycerol fatty acid ester having an HLB of from 9 to 20, preferably a polyglycerol fatty acid ester having an HLB of from 10 to 18, and more preferably a polyglycerol fatty acid ester having an HLB of from 12 to 16.
<11> The cosmetic composition according to any of <1> to <10>, wherein the component (B) is one or two or more selected from the group consisting of polyglyceryl-10 myristate, polyglyceryl-10 dimyristate, polyglyceryl-10 stearate, polyglyceryl-5 stearate, polyglyceryl-6 myristate, and polyglyceryl-6 stearate.
<12> The cosmetic composition according to any of <1> to <11>, wherein a content of the component (B) in the cosmetic composition is 0.3 mass% or more, and preferably 0.5 mass% or more, and is 3 mass% or less, and preferably 1 mass% or less.
<13> The cosmetic composition according to any of <1> to <12>, wherein the content of the component (B) in the cosmetic composition is from 0.3 to 3 mass%, preferably from 0.3 to 1 mass%, and more preferably from 0.5 to 1 mass%.
<14> The cosmetic composition according to any of <1> to <13>, wherein the component (C) is a di- to tetrahydric alcohol.
<15> The cosmetic composition according to any of <1> to <14>, wherein the component (C) is preferably one or two or more selected from the group consisting of ethylene glycol, diethylene glycol, triethylene glycol, polyethylene glycol (having average molecular weight of less than 650), propylene glycol, dipropylene glycol, polypropylene glycol (having average molecular weight of less than 650), isoprene glycol, 1,3-butylene glycol, glycerin, diglycerin, polyglycerin and pentaerythritol; more preferably one or two or more selected from the group consisting of ethylene glycol, diethylene glycol, propylene glycol, dipropylene glycol, 1,3-butylene glycol, glycerin, diglycerin and pentaerythritol; and even more preferably one or two or more selected from the group consisting of dipropylene glycol, 1,3-butylene glycol, glycerin and diglycerin.
<16> The cosmetic composition according to any of <1> to <15>, wherein a content of the component (C) in the cosmetic composition is 13 mass% or more, and preferably 16 mass% or more, and is 31 mass% or less, and preferably 27 mass% or less.
<17> The cosmetic composition according to any of <1> to <16>, wherein the content of the component (C) in the cosmetic composition is from 13 to 31 mass%, and preferably from 16 to 27 mass%.
<18> The cosmetic composition according to any of <1> to <17>, wherein the component (D) is an oil agent having a melting point of 30°C or lower, and preferably one or two or more selected from the group consisting of a hydrocarbon oil, a triglyceride, a fatty acid, an ester oil, a branched or unsaturated higher alcohol, and a silicone.
<19> The cosmetic composition according to any of <1> to <18>, wherein the component (D) is a liquid oil comprising from 70 to 100 mass%, preferably from 75 to 100 mass% of a fatty acid triglyceride having at least one fatty acid group having 5 to 22 carbon atoms in the component (D).
<20> The cosmetic composition according to any of <1> to <19>, wherein the component (D) is a liquid oil comprising 70 mass% or more, based on the component (D), of a fatty acid triglyceride having at least one fatty acid group having 5 to 22 carbon atoms, preferably a liquid oil comprising 70 mass% or more of a fatty acid triglyceride having at least two fatty acid groups having 5 to 22 carbon atoms, and even more preferably a liquid oil comprising 70 mass% or more of a fatty acid triglyceride having three fatty acid groups having 5 to 22 carbon atoms.
<21> The cosmetic composition according to any of <1> to <20>, wherein a content of the component (D) in the cosmetic composition is2 mass% or more, and preferably 5 mass% or more, and is 18 mass% or less, and preferably 15 mass% or less.
<22> The cosmetic composition according to any of <1> to <21>, wherein the content of the component (D) in the cosmetic composition is from 2 to 18 mass%, and preferably from 5 to 15 mass%.
<23> The cosmetic composition according to any of <1> to <22>, further comprising a solid oil having a melting point higher than 30°C as a component (F), wherein a content thereof is preferably 25 mass% or less, more preferably from 0 to 20 mass%, even more preferably from 0 to 10 mass%, and even more preferably from 0 to 5 mass% based on the total amount of the liquid oil (D).
<24> The cosmetic composition according to any of <1> to <23>, wherein the component (E) is one or two or more selected from the group consisting of dihydroxyacetone, glyceraldehyde, 2,3-dihydroxy-succindialdehyde, 2,3-dimethoxysuccindialdehyde, erythrulose, erythrose, 2-amino-3-hydroxy-succindialdehyde, and 2-benzylamino-3-hydroxy-succindialdehyde; preferably one or two or more selected from the group consisting of dihydroxyacetone and erythrulose; and more preferably dihydroxyacetone and erythrulose.
<25> The cosmetic composition according to any of <1> to <24>, wherein a content of the component (E) in the cosmetic composition is 2 mass% or more, and preferably 3 mass% or more, and is 8 mass% or less, and preferably 5 mass% or less.
<26> The cosmetic composition according to any of <1> to <25>, wherein the content of the component (E) in the cosmetic composition is from 2 to 8 mass, preferably from 2 to 5 mass%, and more preferably from 3 to 5 mass%.
<27> The cosmetic composition according to any of <1> to <26>, further comprising a polymer other than the component (A); preferably one or two or more selected from the group consisting of xanthan gum, carrageenan, locust bean gum, guar gum and tamarind seed gum, as a polymer other than the component (A); more preferably one or two or more selected from the group consisting of xanthan gum, locust bean gum and guar gum, as a polymer other than the component (A); and even more preferably xanthan gum, as a polymer other than the component (A).
<28> The cosmetic composition according to <27>, wherein a content of the polymer other than the component (A) is preferably 0.01 mass% or more, more preferably 0.1 mass% or more, and even more preferably 0.15 mass% or more, and is preferably 5 mass% or less, more preferably 3 mass% or less, even more preferably 2 mass% or less, and even more preferably 1 mass% or less.
<29> The cosmetic composition according to <27> or <28>, wherein the content of the polymer other than the component (A) is preferably from 0.01 to 5 mass%, more preferably from 0.1 to 3 mass%, even more preferably from 0.15 to 2 mass%, and even more preferably from 0.15 to 1 mass%.
<30> The cosmetic composition according to any of <1> to <29>, wherein the cosmetic composition has a viscosity of 3,000 mPa·s or higher, and preferably 4,000 mPa·s or higher, and has a viscosity of 12,000 mPa·s or lower, and preferably 9,000 mPa·s or lower.
<31> The cosmetic composition according to any of <1> to <30>, wherein the cosmetic composition has a viscosity of from 3,000 to 12,000 mPa·s, and preferably from 4,000 to 9,000 mPa·s.
<32> The cosmetic composition according to any of <1> to <31>, wherein the cosmetic composition preferably has a pH of 7 or less, more preferably 6 or less, even more preferably 5 or less, and even more preferably 4 or less, and preferably has a pH of 3 or more.
<33> The cosmetic composition according to any of <1> to <32>, wherein the cosmetic composition preferably has a pH of from 3 to 6, more preferably from 3 to 5, and even more preferably from 3 to 4.
<34> The cosmetic composition according to any of <1> to <33>, wherein the cosmetic composition is an emulsified composition, preferably an oil-in-water emulsified composition.
<35> The cosmetic composition according to <34>, wherein a mass ratio of an oil phase to a water phase (oil phase/water phase) in the emulsified composition is preferably 0.01 or more, and more preferably 0.05 or more, and is preferably 0.4 or less, and more preferably 0.2 or less.
<36> The cosmetic composition according to <34> or <35>, wherein the mass ratio of the oil phase to the water phase (oil phase/water phase) in the emulsified composition is preferably from 0.01 to 0.4, and more preferably from 0.05 to 0.2.
<37> The cosmetic composition according to any of <34> to <36>, wherein a water content in the emulsified composition is preferably 50 mass% or more, and more preferably 55 mass% or more, and is preferably 80 mass% or less, and more preferably 75 mass% or less, based on the whole composition.
<38> The cosmetic composition according to any of <34> to <37>, wherein the water content in the emulsified composition is preferably from 50 to 80 mass%, and more preferably from 55 to 75 mass%, based on the whole composition.
<39> Use of the cosmetic composition according to any of <1> to <38> as a self-tanning cosmetic composition.
<40> A method for adjusting a color tone of the skin, comprising applying the cosmetic composition according to any of <1> to <38> to the skin.

### [Examples]

The present invention will be described by way of Examples and Comparative Examples below. The present invention is not limited thereby.

### Examples 1 to 22, Comparative Examples 1 to 13

The test methods of physical property test and the sensory test shown in Examples and Comparative Examples are as described below. The results are described in Tables 1 to 3. It should be noted that each of the contents in the composition is in mass% in the following tables.

### (1) Tanning Speed Test

The sample was applied to the skin in an amount of 2 mg/cm², and the color of the skin was measured from the time immediately after the application till 24 hours after the application, followed by evaluating the change of brightness compared to that before the application. Specifically, CM-2600d manufactured by Konica Minolta, Inc. was used as a colorimeter, and when the absolute value of the difference between the L* value before the application and the L* value after the application (ΔL* value) became 2 or more, it was judged that a tanning effect was exhibited, followed by evaluating the tanning speed on the following criteria.

### Criteria for Evaluating Tanning Speed

A: the tanning effect is observed at 0 to 4 hours after the application
B: the tanning effect is observed at more than 4 hours to 7 hours after the application
C: the tanning effect is observed at more than 7 hours after the application
Z: the tanning effect is not observed at 24 hours after the application

### (2) Test for Evenness of Tanning

When the absolute value of the ΔL* value became 2 or more in the tanning speed test described above, the evenness of tanning was evaluated on a scale of 0 to 4 by visual observation by five specialized panelists, followed by calculating the average point of the evaluation. The evaluation criteria are as described below. For a sample by which a tanning effect was not observed at 24 hours after the application, the evenness of tanning thereof was not evaluated.

### Criteria for Evaluating Evenness of Tanning

4: very even (there are not any parts which appear mottled at all)
3: even (there is a part which partially appears slightly mottled)
2: somewhat even (the whole appears slightly mottled)
1: not even (there is a part which partially appears obviously mottled)
0: not even at all (the whole appears obviously mottled)

### (3) Storage Stability Test

The appearance of the sample after storage at 40°C for six months was visually observed, and the evaluation of storage stability was conducted on the following evaluation criteria.

### Criteria for Evaluating Storage Stability

A: there is no problem of storage stability
B: a little change in physical property is observed
Y: an obvious change in physical property is observed
Z: a phenomenon such as separation or creaming is observed

**[Table 1]**

| | | Components Incorporated | Examples | | | | | | | | | | |
|---|---|---|---|---|---|---|---|---|---|---|---|---|---|
| | | | 1 | 2 | 3 | 4 | 5 | 6* | 7 | 8* | 9 | 10 | 11* |
| 1 | A | (Hydroxyethyl acrylate/sodium acryloyldimethyltaurate) copolymer *1 | 1 | 0.5 | 1.5 | 1 | 1 | 1 | 1 | 1 | 1 | 1 | 1 |
| | A | (Sodium acrylate/sodium acryloyldimethyltaurate) copolymer *2 | | | | | | | | | | | |
| | A | Polyacrylamide *3 | | | | | | | | | | | |
| 2 | | Ammonium acryloyldimethyltaurate/VP copolymer *4 | | | | | | | | | | | |
| 3 | | Xanthan gum | 0.2 | 0.2 | 0.2 | 0.2 | 0.2 | 0.2 | 0.2 | 0.2 | 0.2 | 0.2 | 0.2 |
| 4 | B | Polyglyceryl-10 myristate *5 | 0.5 | 0.5 | 0.5 | 0.5 | 0.5 | 0.5 | 0.5 | 0.5 | 0.5 | | 0.5 |
| 5 | B | Polyglyceryl-10 stearate *6 | | | | | | | | | | 0.5 | |
| 6 | | PEG-60 hydrogenated castor oil *7 | | | | | | | | | | | |
| 7 | C | Glycerin | 4 | 4 | 4 | 2 | 15 | 4 | 4 | 4 | 4 | 4 | 4 |
| 8 | C | Diglycerin | 4 | 4 | 4 | 2 | 5 | 4 | 4 | 4 | 4 | 4 | 4 |
| 9 | C | 1,3-BG | 12 | 12 | 12 | 8 | 10 | 12 | 12 | 12 | 12 | 12 | 12 |
| 10 | D | Glyceryl tri(caprylate/caprate) *8 | 10 | 10 | 10 | 10 | 10 | | 8 | 5 | 10 | 10 | 9 |
| | D | Olive oil | | | | | | | | | | | |
| | D | Macadamia nut oil | | | | | | | | | | | |
| 11 | D | Dicaprylyl carbonate *9 | | | | | | 10 | 2 | 5 | | | |
| 12 | F | Beeswax | | | | | | | | | | | 1 |
| 13 | E | Dihydroxyacetone | 3 | 3 | 3 | 3 | 3 | 3 | 3 | 3 | 3 | 3 | 3 |
| 14 | E | Erythrulose | 2 | 2 | 2 | 2 | 2 | 2 | 2 | 2 | | 2 | 2 |
| 15 | | pH adjuster, in an amount for adjusting pH to 3.5 | q.s. | q.s. | q.s. | q.s. | q.s. | q.s. | q.s. | q.s. | q.s. | q.s. | q.s. |
| 16 | | Purified water | balance | balance | balance | balance | balance | balance | balance | balance | balance | balance | balance |
| Evaluation result | | Viscosity (m·Pas) | 6000 | 3000 | 12000 | 5000 | 6000 | 3500 | 6000 | 6000 | 6000 | 10000 | 13000 |
| | | Storage stability | A | B | A | B | A | B | B | Y | A | A | A |
| | | Tanning Speed | A | A | B | A | B | C | A | A | A | B | C |
| | | Evenness of Tanning | 3.6 | 3.4 | 3.2 | 3.2 | 3.4 | 3.3 | 3.6 | 3.5 | 3 | 3.5 | 3.1 |

| | | | | | | | | | | | | | |
|---|---|---|---|---|---|---|---|---|---|---|---|---|---|
| * Reference Examples *1 SEPINOV EMT 10 (manufactured by SEPPIC) *2 SIMULGEL EG (manufactured by SEPPIC) *3 SEPIGEL 305 (manufactured by SEPPIC) *4 ARISTFLEX-AVC (manufactured by Clariant) *5 S face M1001 (manufactured by Sakamoto Yakuhin Kogyo Co., Ltd.) *6 Sunsoft Q18Y-C (manufactured by Taiyo Kagaku Co., Ltd.) *7 Nikkol HCO-60 (manufactured by Nihon Surfactant Kogyo K.K.) *8 Myritol 318 (manufactured by BASF SE) *9 Cetiol CC (manufactured by BASF SE) | | | | | | | | | | | | | |

**[Table 2]**

| | | Components Incorporated | Examples | | | | | | | | | | |
|---|---|---|---|---|---|---|---|---|---|---|---|---|---|
| | | | 12 | 13 | 14 | 15 | 16* | 17 | 18 | 19* | 20 | 21 | 22* |
| 1 | A | (Hydroxyethyl acrylate/sodium acryloyldimethyltaurate) copolymer *1 | | | 1 | 1 | 1 | 1 | 1 | 1 | 1 | 1 | 1 |
| | A | (Sodium acrylate/sodium acryloyldimethyltaurate) copolymer *2 | 1 | | | | | | | | | | |
| | A | Polyacrylamide *3 | | 1 | | | | | | | | | |
| 2 | | (Ammonium acryloyldimethyltaurate/VP) copolymer *4 | | | | | | | | | | | |
| 3 | | Xanthan gum | 0.2 | 0.2 | 0.2 | 0.2 | 0.2 | 0.2 | 0.2 | 0.2 | 0.2 | 0.2 | 0.2 |
| 4 | B | Polyglyceryl-10 myristate *5 | 0.5 | 0.5 | 0.5 | 0.5 | 0.5 | 0.5 | 0.5 | 0.1 | 1 | 3 | 6 |
| 5 | B | Polyglyceryl-10 stearate *6 | | | | | | | | | | | |
| 6 | | PEG-60 hydrogenated castor oil *7 | | | | | | | | | | | |
| 7 | C | Glycerin | 4 | 4 | 4 | 4 | 4 | 4 | 4 | 4 | 4 | 4 | 4 |
| 8 | C | Diglycerin | 4 | 4 | 4 | 4 | 4 | 4 | 4 | 4 | 4 | 4 | 4 |
| 9 | C | 1,3-BG | 12 | 12 | 12 | 12 | 12 | 12 | 12 | 12 | 12 | 12 | 12 |
| 10 | D | Glyceryl tri(caprylate/caprate) *8 | 10 | 10 | | | 1 | 5 | 15 | 10 | 10 | 10 | 10 |
| | D | Olive oil | | | 10 | | | | | | | | |
| | D | Macadamia nut oil | | | | 10 | | | | | | | |
| 11 | D | Dicaprylyl carbonate *9 | | | | | | | | | | | |
| 12 | F | Beeswax | | | | | | | | | | | |
| 13 | E | Dihydroxyacetone | 3 | 3 | 3 | 3 | 3 | 3 | 3 | 3 | 3 | 3 | 3 |
| 14 | E | Erythrulose | 2 | 2 | 2 | 2 | 2 | 2 | 2 | 2 | 2 | 2 | 2 |
| 15 | | pH adjuster, in an amount for adjusting pH to 3.5 | q.s. | q.s. | q.s. | q.s. | q.s. | q.s. | q.s. | q.s. | q.s. | q.s. | q.s. |
| 16 | | Purified water | balance | balance | balance | balance | balance | balance | balance | balance | balance | balance | balance |
| Evaluation result | | Viscosity (m·Pas) | 5000 | 6500 | 6000 | 7000 | 3000 | 5000 | 6000 | 4000 | 6000 | 4000 | |
| | | Storage stability | A | A | A | A | Y | A | A | Y | A | A | |
| | | Tanning Speed | A | A | A | A | B | A | A | A | A | A | |
| | | Evenness of Tanning | 3.4 | 3.6 | 3.5 | 3.6 | 3.1 | 3.3 | 3.5 | 3 | 3.5 | 3.3 | |

| | | | | | | | | | | | | | |
|---|---|---|---|---|---|---|---|---|---|---|---|---|---|
| * Reference Examples *1 SEPINOV EMT 10 (manufactured by SEPPIC) *2 SIMULGEL EG (manufactured by SEPPIC) *3 SEPIGEL 305 (manufactured by SEPPIC) *4 ARISTFLEX-AVC (manufactured by Clariant) *5 S face M1001 (manufactured by Sakamoto Yakuhin Kogyo Co., Ltd.) *6 Sunsoft Q18Y-C (manufactured by Taiyo Kagaku Co., Ltd.) *7 Nikkol HCO-60 (manufactured by Nihon Surfactant Kogyo K.K.) *8 Myritol 318 (manufactured by BASF SE) *9 Cetiol CC (manufactured by BASF SE) | | | | | | | | | | | | | |

**[Table 3]**

| | | Components Incorporated | Comparative Examples | | | | | | | | | | | | |
|---|---|---|---|---|---|---|---|---|---|---|---|---|---|---|---|
| | | | 1 | 2 | 3 | 4 | 5 | 6 | 7 | 8 | 9 | 10 | 11 | 12 | 13 |
| 1 | A | (Hydroxyethyl acrylate/sodium acryloyldimethyltaurate) copolymer *1 | 0.25 | 2.5 | | | 1 | 1 | 1 | 1 | 1 | 1 | 1 | 1 | 1 |
| 2 | | (Ammonium acryloyldimethyltaurate/VP) copolymer *2 | | | 1 | | | | | | | | | | |
| | | Carboxyvinyl polymer K | | | | 1 | | | | | | | | | |
| 3 | | Xanthan gum | | 0.3 | 0.5 | 0.5 | 0.2 | 0.2 | 0.2 | 0.2 | 0.2 | 0.2 | 0.2 | 0.2 | 0.2 |
| 4 | B | Polyglyceryl-10 myristate *3 | 0.5 | 0.5 | 0.5 | 0.5 | 0.5 | 0.5 | 0.5 | 0.5 | | | | | |
| 5 | B | Polyglyceryl-10 stearate *4 | | | | | | | | | | | | | |
| 6 | | PEG-60 hydrogenated castor oil *5 | | | | | | | | | 0.5 | | | | |
| | | Glyceryl monostearate (SE) | | | | | | | | | | 0.5 | | | |
| | | Polyethylene glycol(45) monostearate *6 | | | | | | | | | | | 0.5 | | |
| | | Polyoxyethylene (20) sorbitan monolaurate *7 | | | | | | | | | | | | 0.5 | |
| | | Polyoxyethylene(2) stearyl ether *8 | | | | | | | | | | | | | 0.5 |
| 7 | C | Glycerin | 4 | 4 | 4 | 4 | 1 | 8 | 4 | 4 | 4 | 4 | 4 | 4 | 4 |
| 8 | C | Diglycerin | 4 | 4 | 4 | 4 | 1 | 8 | 4 | 4 | 4 | 4 | 4 | 4 | 4 |
| 9 | C | 1,3-BG | 12 | 12 | 12 | 12 | 4 | 24 | 12 | 12 | 12 | 12 | 12 | 12 | 12 |
| 10 | D | Glyceryl tri(caprylate/caprate) *9 | 10 | 10 | 10 | 10 | 10 | 10 | | 30 | 10 | 10 | 10 | 10 | 10 |
| 11 | D | Dicaprylyl carbonate *10 | | | | | | | | | | | | | |
| 12 | F | Beeswax | | | | | | | 1 | | | | | | |
| 13 | E | Dihydroxyacetone | 3 | 3 | 3 | 3 | 3 | 3 | 3 | 3 | 3 | 3 | 3 | 3 | 3 |
| 14 | E | Erythrulose | 2 | 2 | 2 | 2 | 2 | 2 | 2 | 2 | 2 | 2 | 2 | 2 | 2 |
| 15 | | pH adjuster, in an amount for adjusting pH to 3.5 | q.s. | q.s. | q.s. | q.s. | q.s. | q.s. | q.s. | q.s. | q.s. | q.s. | q.s. | q.s. | q.s. |
| 16 | | Purified water | balance | balance | balance | balance | balance | balance | balance | balance | balance | balance | balance | balance | balance |
| Evaluation result | | Viscosity (m·Pas) | 750 | 35000 | 8000 | 3500 | 5000 | 7000 | 13000 | 7000 | 10000 | 15000 | 13000 | 5000 | 14000 |
| | | Storage stability | Z | A | Z | Z | Y | A | Z | Z | Z | A | B | Z | B |
| | | Tanning Speed | A | C | C | B | B | Z | C | B | B | C | C | B | C |
| | | Evenness of Tanning | 2.5 | 2.8 | 2.1 | 2.5 | 2.8 | - | 2 | 2.8 | 3.2 | 3.0 | 3.1 | 2.7 | 2.9 |

| | | | | | | | | | | | | | | | |
|---|---|---|---|---|---|---|---|---|---|---|---|---|---|---|---|
| *1 SEPINOV EMT 10 (manufactured by SEPPIC) *2 ARISTFLEX-AVC (manufactured by Clariant) *3 S face M1001 (manufactured by Sakamoto Yakuhin Kogyo Co., Ltd.) *4 Sunsoft Q18Y-C (manufactured by Taiyo Kagaku Co., Ltd.) *5 Nikkol HCO-60 (manufactured by Nihon Surfactant Kogyo K.K.) *6 Nikkol MYS-45V (manufactured by Nihon Surfactant Kogyo K.K.) *7 REODOL L-120 (manufactured by Kao Corporation) *8 BRIJ S2 (manufactured by Croda) *9 Myritol 318 (manufactured by BASF SE) *10 Cetiol CC (manufactured by BASF SE) | | | | | | | | | | | | | | | |

### <Examples: Production Method>

(1) Stir and mix the components 1 to 11, 19 and 20 uniformly at 25°C (the water phase 1) .
(2) Stir and mix the components 12 to 16 uniformly at 25°C (the oil phase 1).
(3) Add the oil phase 1 obtained in the step (2) at 25°C stepwise to the water phase 1 obtained in the step (1), and stir and mix the resultant uniformly with a homogenizer (the emulsion 1).
(4) Add the components 17 and 18 at 25°C to the emulsion 1 obtained in the step (3), and stir and mix the resultant uniformly with a homogenizer.

### <Comparative Examples: Production Method>

(1) Stir and mix the components 1 to 14, 20 and 21 uniformly at 25°C (the water phase 1).
(2) Stir and mix the components 15 to 17 uniformly at 25°C (the oil phase 1).
(3) Add the oil phase 1 obtained in the step (2) at 25°C stepwise to the water phase 1 obtained in the step (1), and stir and mix the resultant uniformly with a homogenizer (the emulsion 1).
(4) Add the components 18 and 19 to the emulsion 1 obtained in the step (3) at 25°C, and stir and mix the resultant uniformly with a homogenizer.

As is clear from Tables 1 to 3, the cosmetic compositions of the present invention comprising the components (A) to (E) and having a viscosity adjusted to from 3,000 to 12,000 mPa·s were excellent in all of storage stability, tanning speed and evenness of tanning. In contrast, the cosmetic composition of Comparative Example 1, which had a lower viscosity than the viscosity range according to the present invention, exhibited poor storage stability and was inferior in evenness of tanning. The cosmetic composition of Comparative Example 2, which had a higher viscosity than the viscosity range according to the present invention, was inferior in tanning speed and evenness of tanning. The cosmetic compositions of Comparative Examples 3 and 4, which did not contain the component (A), exhibited poor storage stability and were inferior in evenness of tanning. The cosmetic compositions of Comparative Examples 5 and 6, in which the content of the component (C) was outside of the range according to the present invention, exhibited decreased storage stability or a decreased tanning speed, and were also inferior in evenness of tanning. The cosmetic compositions of Comparative Examples 7 and 8, which contained no or excessive component (D), respectively, exhibited poor storage stability and were inferior in evenness of tanning. The cosmetic compositions of Comparative Examples 9, 10 and 12, which did not contain the component (B), exhibited poor storage stability, and the cosmetic compositions of Comparative Examples 11 and 13, which did not contain the component (B), had a higher viscosity and therefore were inferior in evenness of tanning.

### Example 23: Self-Tanning Cosmetic Composition (Oil-in-Water Emulsion)

**[Table 4]**

| | (mass%) |
|---|---|
| Red No. 504 | 0.0001 |
| Yellow No. 4 | 0.001 |
| EDTA-2Na | 0.05 |
| Glycerin | 4 |
| Diglycerin | 4 |
| 1,3-Butylene glycol | 15 |
| Dihydroxyacetone | 3 |
| Erythrulose | 3 |
| Xanthan gum | 0.2 |
| (Hydroxyethyl acrylate/sodium acryloyldimethyltaurate) copolymer (SEPINOV EMT 10, manufactured by SEPPIC) | 1.0 |
| Polyacrylamide mixture (SEPIGEL 305, manufactured by SEPPIC) | 0.5 |
| Polyglyceryl-10 myristate (S face M1001, manufactured by Sakamoto Yakuhin Kogyo Co., Ltd. ) | 0.5 |
| Glyceryl tri(caprylate/caprate) (Myritol 318, manufactured by BASF SE) | 10 |
| Phenoxyethanol | 0.1 |
| Citric acid | 0.05 |
| Sodium citrate | 0.05 |
| Sodium pyrosulfite | 0.2 |
| Dipotassium glycyrrhizinate | 0.1 |
| Silk extract | 0.1 |
| Fruit extract mixture (Multifruit BSC, manufactured by Arch Personal care Products L.P.) | 0.1 |
| Perfume | 0.2 |
| Purified water | balance |

As a cosmetic composition having the composition described above was prepared and evaluated, it exhibited good storage stability and had excellent tanning performance.

The composition of the perfume used in Example 23 described above is shown in Table 5.

**[Table 5]**

| Mixed floral perfume | |
|---|---|
| (Components) | (mass%) |
| Bergamot oil | 2.0 |
| Linalyl acetate | 3.5 |
| Methyl anthranilate | 0.2 |
| Petitgrain oil | 0.5 |
| Aurantiol 10% DPG | 1.0 |
| Amyl allyl glycolate 1% DPG | 0.5 |
| Galbanum oil 1% DPG | 0.1 |
| Blackcurrant bud absolute 10% DPG | 1.5 |
| Tagetes oil 10% DPG | 0.8 |
| Ylang ylang oil extra | 2.0 |
| Benzyl acetate | 5.0 |
| Methyl dihydrojasmonate | 15.0 |
| cis-Jasmon 10% DPG | 2.0 |
| Jasmin absolute | 0.5 |
| Indol 5% DPG | 0.5 |
| α-Hexyl cinnamaldehyde | 1.5 |
| L-Citronellol | 0.5 |
| Rose oil | 0.5 |
| Rose absolute | 0.5 |
| Damascenones 1% DPG | 0.5 |
| L-Rose oxide 1% DPG | 0.5 |
| Dimethylbenzylcarbonyl acetate | 1.0 |
| Hydroxycitronellal | 3.0 |
| Lyral | 4.5 |
| Cyclamen aldehyde | 0.5 |
| α-isomethylionone | 4.0 |
| Orris concrete 10% DPG | 0.8 |
| Methyleugenol | 0.5 |
| Iso E super | 2.5 |
| Vertofix coeur | 4.0 |
| Vetiver acetate | 2.0 |
| Sandalwood oil | 1.5 |
| Bacdanol 10% DPG | 1.0 |
| Patchouli oil 10% DPG | 0.2 |
| Evernyl 10% DPG | 1.5 |
| Galaxolide 50% benzyl benzoate | 12.0 |
| Cyclopentadecanolide | 4.0 |
| Heliotropine | 0.5 |
| Coumarin | 0.5 |
| Vanillin 10% | 0.5 |
| Ethylvanillin 10% | 2.5 |
| Raspberry ketone 10% | 0.5 |
| γ-Undecalactone 10% | 1.5 |
| γ-decalactone 10% | 1.5 |
| Labdanum absolute 10% | 0.5 |
| Dipropylene glycol | balance |
| Total | 100.0 |

### [Industrial Applicability]

According to the present invention, a cosmetic composition can be provided which has good storage stability and high moisturizing property and can develop quickly tanned skin having natural tint.

## Claims

1. A cosmetic composition comprising the following components (A) to (E):
(A) 0.3 to 1.5 mass% of a copolymer or crosspolymer which comprises, as a constituent unit, a combination of either an acrylic acid salt and 2-acrylamido-2-methylpropanesulfonic acid or a salt thereof, or of a hydroxyethyl acrylate and 2-acrylamido-2-methylpropanesulfonic acid or a salt thereof; polyacrylamide; or a mixture thereof;
(B) 0.3 to 3 mass% of a polyglycerol fatty acid ester;
(C) 13 to 31 mass% of a polyhydric alcohol;
(D) 2 to 18 mass% of a liquid oil having a melting point of 30°C or lower; and
(E) 2 to 8 mass% of a tanning agent,
wherein the component (D) comprises 70 mass% or more of a fatty acid triglyceride having at least one fatty acid group having 5 to 22 carbon atoms;
and having a viscosity of from 3,000 to 12,000 mPa·s.

2. The cosmetic composition according to claim 1, wherein the component (B) is an ester of polyglycerin having a condensation degree of from 2 to 10 and a saturated or unsaturated fatty acid having 12 to 22 carbon atoms, and has an esterification degree of from 20 to 75%.

3. The cosmetic composition according to claims 1 or 2, wherein the component (B) is an ester of polyglycerin having a condensation degree of from 6 to 10 and a saturated or unsaturated fatty acid having 14 to 18 carbon atoms, and has an esterification degree of from 20 to 75%.

4. The cosmetic composition according to any one of claims 1 to 3, wherein the component (B) is a polyglycerol fatty acid ester having an HLB of from 9 to 20.

5. The cosmetic composition according to any one of claims 1 to 4, wherein the component (B) is a polyglycerol fatty acid ester having an HLB of from 10 to 18.

6. The cosmetic composition according to any one of claims 1 to 5, wherein the component (B) is one or two or more selected from the group consisting of polyglyceryl-10 myristate, polyglyceryl-10 dimyristate, polyglyceryl-10 stearate, and polyglyceryl-6 myristate.

7. The cosmetic composition according to any one of claims 1 to 6, wherein the component (A) is one or two or more selected from the group consisting of (hydroxyethyl acrylate/sodium acryloyldimethyltaurate) copolymer, (sodium acrylate/sodium acryloyldimethyltaurate) copolymer, and polyacrylamide.

8. The cosmetic composition according to any one of claims 1 to 7, wherein the component (D) is a liquid oil comprising 70 mass% or more of a fatty acid triglyceride having at least two fatty acid groups having 5 to 22 carbon atoms.

9. The cosmetic composition according to any one of claims 1 to 8, wherein the component (E) is dihydroxyacetone and erythrulose.

10. The cosmetic composition according to any one of claims 1 to 9, further comprising (F) a solid oil having a melting point higher than 30°C, wherein a content of the component (F) is 25 mass% or less based on the total amount of the component (D).

11. The cosmetic composition according to any one of claims 1 to 10, wherein a content of a polymer other than the component (A) is from 0.01 to 5 mass%.

12. The cosmetic composition according to any one of claims 1 to 11, which has a pH of 7 or less.

13. Use of the cosmetic composition according to any one of claims 1 to 12 as a self-tanning cosmetic composition.

14. A method for adjusting a color tone of the skin, comprising topically applying the cosmetic composition according to any one of claims 1 to 12 to the skin.

## Patentansprüche

1. Kosmetische Zusammensetzung umfassend die folgenden Komponenten (A) bis (E):
(A) 0,3 bis 1,5 Gew.% eines Copolymers oder Kreuzpolymers, das als Wiederholungseinheit eine Kombination von entweder einem Acrylsäuresalz und 2-Acrylamido-2-methylpropansulfonsäure oder einem Salz davon oder von einem Hydroxyethylacrylat und 2-Acrylamido-2-methylpropansulfonsäure oder einem Salz davon; Polyacrylamid; oder eine Mischung davon enthält;
(B) 0,3 bis 3 Gew.% eines Polyglycerolfettsäureesters;
(C) 13 bis 31 Gew.% eines mehrwertigen Alkohols;
(D) 2 bis 18 Gew.% eines flüssigen Öls mit einem Schmelzpunkt von 30 °C oder niedriger; und
(E) 2 bis 8 Gew.% eines Bräunungsmittels;
wobei die Komponente (D) 70 Gew.% oder mehr eines Fettsäuretriglycerids mit mindestens einer Fettsäuregruppe mit 5 bis 22 Kohlenstoffatomen umfasst;
und wobei die Viskosität von 3.000 bis 12.000 mPa·s beträgt.

2. Kosmetische Zusammensetzung gemäß Anspruch 1, wobei die Komponente (B) ein Ester eines Polyglycerins mit einem Kondensationsgrad von 2 bis 10 und einer gesättigten oder ungesättigten Fettsäure mit 12 bis 22 Kohlenstoffatomen ist und einen Veresterungsgrad von 20 bis 75% aufweist.

3. Kosmetische Zusammensetzung gemäß Anspruch 1 oder 2, wobei die Komponente (B) ein Ester eines Polyglycerins mit einem Kondensationsgrad von 6 bis 10 und einer gesättigten oder ungesättigten Fettsäure mit 14 bis 18 Kohlenstoffatomen ist und einen Veresterungsgrad von 20 bis 75% aufweist.

4. Kosmetische Zusammensetzung gemäß einem der Ansprüche 1 bis 3, wobei die Komponente (B) ein Polyglycerolfettsäureester mit einem HLB-Wert von 9 bis 20 ist.

5. Kosmetische Zusammensetzung gemäß einem der Ansprüche 1 bis 4, wobei die Komponente (B) ein Polyglycerolfettsäureester mit einem HLB-Wert von 10 bis 18 ist.

6. Kosmetische Zusammensetzung gemäß einem der Ansprüche 1 bis 5, wobei die Komponente (B) eine oder zwei oder mehrere ausgewählt aus der Gruppe bestehend aus Polyglycerol-10 Myristat, Polyglycerol-10 Dimyristat, Polyglycerol-10 Stearat und Polyglycerol-6 Myristat ist.

7. Kosmetische Zusammensetzung gemäß einem der Ansprüche 1 bis 6, wobei die Komponente (A) eine oder zwei oder mehrere ausgewählt aus der Gruppe bestehend aus (Hydroxyethylacrylat/Natriumacryloyldimethyltaurat)-Copolymer, (Natriumacrylat/ Natriumacryloyldimethyltaurat)-Copolymer und Polyacrylamid ist.

8. Kosmetische Zusammensetzung gemäß einem der Ansprüche 1 bis 7, wobei die Komponente (D) ein flüssiges Öl ist, das 70 Gew.% oder mehr eines Fettsäuretriglycerids mit mindestens zwei Fettsäuregruppen mit 5 bis 22 Kohlenstoffatomen umfasst.

9. Kosmetische Zusammensetzung gemäß einem der Ansprüche 1 bis 8, wobei die Komponente (E) Dihydroxyaceton und Erythrulose ist.

10. Kosmetische Zusammensetzung gemäß einem der Ansprüche 1 bis 9, des Weiteren umfassend ein festes Öl (F) mit einem Schmelzpunkt, der höher als 30 °C ist, wobei der Gehalt der Komponente (F) 25 Gew.% oder weniger basierend auf der Gesamtmenge der Komponente (D) beträgt.

11. Kosmetische Zusammensetzung gemäß einem der Ansprüche 1 bis 10, wobei ein Gehalt eines anderen Polymers als der Komponente (A) von 0,01 bis 5 Gew.% beträgt.

12. Kosmetische Zusammensetzung gemäß einem der Ansprüche 1 bis 11, die einen pH-Wert von 7 oder weniger aufweist.

13. Verwendung der kosmetischen Zusammensetzung gemäß einem der Ansprüche 1 bis 12 als selbstbräunende kosmetische Zusammensetzung.

14. Verfahren zur Einstellung des Farbtons der Haut, umfassend das lokale Aufbringen der kosmetischen Zusammensetzung gemäß einem der Ansprüche 1 bis 12 auf die Haut.

## Revendications

1. Composition cosmétique comprenant les composants (A) à (E) suivants :
(A) 0,3 à 1,5 % en masse d'un copolymère ou d'un polymère réticulé qui comprend, comme unité constituante, une combinaison d'un sel d'acide acrylique et d'acide 2-acrylamido-2-méthylpropanesulfonique ou d'un sel de celui-ci, ou d'un acrylate d'hydroxyéthyle et d'acide 2-acrylamido-2-méthylpropanesulfonique ou d'un sel de celui-ci ; du polyacrylamide ; ou un mélange de ceux-ci ;
(B) 0,3 à 3 % en masse d'un ester d'acide gras de polyglycérol ;
(C) 13 à 31 % en masse d'un alcool polyhydrique ;
(D) 2 à 18 % en masse d'une huile liquide ayant un point de fusion de 30°C ou moins ; et
(E) 2 à 8 % en masse d'un agent bronzant,
dans laquelle le composant (D) comprend 70 % en masse ou plus d'un triglycéride d'acide gras ayant au moins un groupe acide gras ayant 5 à 22 atomes de carbone ; et
ayant une viscosité de 3 000 à 12 000 mPa.s.

2. Composition cosmétique selon la revendication 1, dans laquelle le composant (B) est un ester de polyglycérine ayant un degré de condensation de 2 à 10 et d'acide gras saturé ou insaturé ayant 12 à 22 atomes de carbone, et a un degré d'estérification de 20 à 75 %.

3. Composition cosmétique selon les revendications 1 ou 2, dans laquelle le composant (B) est un ester de polyglycérine ayant un degré de condensation de 6 à 10 et d'un acide gras saturé ou insaturé ayant 14 à 18 atomes de carbone, et a un degré d'estérification de 20 à 75 %.

4. Composition cosmétique selon l'une quelconque des revendications 1 à 3, dans laquelle le composant (B) est un ester d'acide gras et de polyglycérol ayant un équilibre hydrophile-lipophile (HLB) de 9 à 20.

5. Composition cosmétique selon l'une quelconque des revendications 1 à 4, dans laquelle le composant (B) est un ester d'acide gras et de polyglycérol ayant un équilibre hydrophile-lipophile (HLB) de 10 à 18.

6. Composition cosmétique selon l'une quelconque des revendications 1 à 5, dans laquelle le composant (B) est un ou deux composés ou plus choisis dans le groupe constitué du myristate de polyglycéryle-10, du dimyristate de polyglycéryle-10, du stéarate de polyglycéryle-10, et du myristate de polyglycéryle-6.

7. Composition cosmétique selon l'une quelconque des revendications 1 à 6, dans laquelle le composant (A) est un ou deux ou composés ou plus choisis dans le groupe constitué du copolymère d'acrylate d'hydroxyéthyle et d'acryloyldiméthyltaurate de sodium, du copolymère d'acrylate de sodium et d'acryloyldiméthyltaurate de sodium et du polyacrylamide.

8. Composition cosmétique selon l'une quelconque des revendications 1 à 7, dans laquelle le composant (D) est une huile liquide comprenant 70 % en masse ou plus d'un triglycéride d'acide gras ayant au moins deux groupes acide gras ayant 5 à 22 atomes de carbone.

9. Composition cosmétique selon l'une quelconque des revendications 1 à 8, dans laquelle le composant (E) est la dihydroxyacétone et l'érythrulose.

10. Composition cosmétique selon l'une quelconque des revendications 1 à 9, comprenant en outre (F) une huile solide ayant un point de fusion supérieur à 30 °C, dans laquelle une teneur du composant (F) est de 25 % en masse ou moins sur la base de la quantité totale du composant (D).

11. Composition cosmétique selon l'une quelconque des revendications 1 à 10, dans laquelle une teneur du polymère autre que le composant (A) est de 0,01 à 5 % en masse.

12. Composition cosmétique selon l'une quelconque des revendications 1 à 11, qui a un pH de 7 ou moins.

13. Utilisation de la composition cosmétique selon l'une quelconque des revendications 1 à 12 comme composition cosmétique auto-bronzante.

14. Procédé d'ajustement d'une nuance de couleur de la peau, comprenant l'application topique de la composition cosmétique selon l'une quelconque des revendications 1 à 12 à la peau.
